# EUROPÄISCHE PATENTANMELDUNG

(11) **EP 3 058 866 A2**
(43) Veröffentlichungstag der Anmeldung: **24.08.2016**
(21) Anmeldenummer: 16155934.9
(22) Anmeldetag: 16.02.2016
(51) Int. Cl.: A61B 5/00

(54) **VERFAHREN UND VORRICHTUNG ZUR KONTROLLIERTEN ABGABE VON PARTIKELN**

(30) Priorität: 20.02.2015 DE 102015102492
(71) Anmelder: Bluestone Technology GmbH, 55286 Wörrstadt (DE)
(72) Erfinder: Görgen, Frank, 55268 Nieder-Olm (DE); Sehlinger, Torsten, 55278 Undenheim (DE)
(74) Vertreter: Manitz, Finsterwald & Partner GbR

(57) **Zusammenfassung**

Offenbart wird ein Verfahren zur kontrollierten Abgabe von allergenen Partikeln zumindest einer ersten und einer zweiten Art in einen Raum (12), wobei die allergenen Partikel der ersten Art an einer ersten Abgabestelle und die allergenen Partikel der zweiten Art an einer zweiten Abgabestelle in den Raum (12) abgegeben werden, die von der ersten Abgabestelle beabstandet ist, wobei die allergenen Partikel der ersten Art ausschließlich in einen ersten Korridor (K1) und die allergenen Partikel der zweiten Art ausschließlich in einen zweiten Korridor (K2) innerhalb des Raumes abgegeben werden, wobei die beiden Korridore zumindest über einen vorgegebenen Erstreckungsbereich (E) nicht überlappen. Offenart wird ferner eine Vorrichtung zur Durchführung des Verfahrens, umfassend: eine insbesondere mobile Kammer (10), in deren Inneren ein Raum (12) vorgesehen ist, eine erste Abgabeeinrichtung (14) für allergene Partikel einer ersten Art und eine zweite Abgabeeinrichtung (16) für allergene Partikel einer zweiten Art, die von der ersten Abgabeeinrichtung (14) beabstandet ist, wobei die Abgabeeinrichtungen (14, 16) im Bereich einer Decke des Raumes (12) angeordnet und so orientiert sind, dass die allergenen Partikel der ersten Art ausschließlich in einen ersten Korridor (K1) und die allergenen Partikel der zweiten Art ausschließlich in einen zweiten Korridor (K2) innerhalb des Raumes (12) abgegeben werden, wobei die beiden Korridore (K1, K2) zumindest über einen vorgegebenen Erstreckungsbereich (E) nicht überlappen.

## Beschreibung

Die vorliegende Erfindung betrifft ein Verfahren und eine Vorrichtung zur kontrollierten Abgabe von allergenen Partikeln, um Patienten bzw. Probanden mit Allergien einer dosierten und kontrollierten Menge an allergenen Partikeln auszusetzen. Indem der Patient unterschiedlichen Allergenen in unterschiedlichen Konzentrationen ausgesetzt wird, kann herausgefunden werden, welche Symptome in welcher Stärke auftreten.

Aus dem Stand der Technik sind verschiedene Verfahren und Vorrichtungen zur Abgabe von allergenen Partikeln bekannt, bei denen die Abgabe der Partikel über einen turbulenten Luftstrom erfolgt, was zu einer diskontinuierlichen und räumlich ungleichmäßigen Verteilung der Partikel führt.

Es ist die Aufgabe der vorliegenden Erfindung, ein Verfahren und eine Vorrichtung zur kontinuierlichen Abgabe von allergenen Partikeln zu schaffen, mit denen Patienten in kurzer Zeit auf verschiedene Allergene getestet werden können.

Die Lösung dieser Aufgabe erfolgt durch die Merkmale der unabhängigen Ansprüche.

Bei dem erfindungsgemäßen Verfahren zur kontrollierten Abgabe von allergenen Partikeln werden allergene Partikel zumindest einer ersten und einer zweiten Art in ein und denselben Raum abgegeben, wobei die allergenen Partikel der ersten Art an einer ersten Abgabestelle und die allergenen Partikel der zweiten Art an einer zweiten Abgabestelle in den Raum abgegeben werden, wobei die beiden Abgabestellen voneinander beabstandet sind. Erfindungsgemäß werden die allergenen Partikel der ersten Art ausschließlich in einen ersten Korridor und die allergenen Partikel der zweiten Art ausschließlich in einen zweiten Korridor innerhalb ein und desselben Raumes abgegeben, wobei die beiden Korridore zumindest über einen vorgegebenen Erstreckungsbereich nicht überlappen.

Das erfindungsgemäße Verfahren beruht auf der Erkenntnis, dass sich innerhalb ein und desselben Raumes durchaus allergene Partikel verschiedener Art gerichtet so abgeben lassen, dass sich diese zumindest innerhalb eines bestimmten Erstreckungsbereichs, ausgehend von der jeweiligen Abgabestelle, nicht überlappen. Außerhalb des vorgegebenen Erstreckungsbereiches kann eine Überlappung der Korridore erfolgen, was unschädlich ist, wenn der Inhalationsbereich des Patienten bzw. der Patienten innerhalb des vorgegebenen Erstreckungsbereichs liegt.

Durch das erfindungsgemäße Verfahren können einerseits verschiedene Patienten innerhalb ein und desselben Raumes verschiedenen allergenen Partikeln ausgesetzt werden. Andererseits kann ein und derselbe Patient innerhalb kürzester Zeit auf unterschiedliche Allergene getestet werden.

Vorteilhafte Ausführungsformen der Erfindung sind in der Beschreibung, der Zeichnung sowie die Unteransprüchen beschrieben.

Nach einer ersten vorteilhaften Ausführungsform können die Korridore, in welche die unterschiedlichen allergenen Partikel abgegeben werden, in vertikaler Richtung nach unten ausgerichtet werden. Hierdurch wird eine gleichmäßige Abgabe der Partikel innerhalb des zugehörigen Korridors begünstigt. In diesem Zusammenhang kann es auch vorteilhaft sein, wenn die Mittelachsen der Korridore im Wesentlichen parallel zueinander ausgerichtet werden, da dies dazu beiträgt, eine Vermischung der unterschiedlichen Partikel so weit wie möglich auszuschließen.

Zur Durchführung des erfindungsgemäßen Verfahrens kann es vorteilhaft sein, wenn in dem Raum zumindest im Bereich der Abgabestellen eine Luftströmung mit einer Vorzugsrichtung erzeugt wird, die in Richtung der Schwerkraft weist. Auf diese Weise werden die allergenen Partikel nach Abgabe in den Raum durch die umgebende Luftströmung nach unten bewegt, ohne dass es zu einer nennenswerten Querbewegung kommt. Vorteilhaft ist hierbei, wenn die Luftströmung als im Wesentlichen laminare Luftströmung erzeugt wird, um eine ungewünschte Vermischung der unterschiedlichen Partikel zu vermeiden.

Zur Erzeugung einer Luftströmung mit nach unten gerichteter Vorzugsrichtung kann Luft in den Raum durch eine Luftzuführung eingebracht werden, die im Bereich der Decke des Raumes verläuft und die insbesondere entlang zweier paralleler Wände des Raums verläuft. Weiterhin kann Luft aus dem Raum durch eine Bodenabsaugung abgesaugt werden, die insbesondere zumindest entlang zweier paralleler Wände des Raums verläuft. Grundsätzlich sind jedoch verschiedenste Anordnungen von Luftzuführung und Luftabsaugung über die Fläche des Raumes möglich, solange gewährleistet ist, dass zumindest im Bereich der Abgabeeinrichtungen eine Luftströmung mit Vorzugsrichtung in Richtung der Schwerkraft erzeugt wird.

Die Partikel können in ihren jeweiligen Korridor mit einer sehr geringen Geschwindigkeit abgegeben werden, so dass sie nur mit Erdbeschleunigung oder aber durch die Luftströmung mit Vorzugsrichtung nach unten in Richtung des Bodens des Raumes beschleunigt werden. Es kann jedoch auch vorteilhaft sein, die Partikel in Richtung der Schwerkraft mit einer Geschwindigkeit in ihren zugehörigen Korridor abzugeben, die größer als die Geschwindigkeit der Luftströmung ist.

Nach einem weiteren Aspekt der vorliegenden Erfindung betrifft diese eine Vorrichtung zur Durchführung eines Verfahrens der vorstehend beschriebenen Art, wobei diese eine insbesondere mobile Kammer umfasst, in deren Inneren ein Raum vorgesehen ist, wobei zumindest eine erste Abgabeeinrichtung für allergene Partikel einer ersten Art und eine zweite Abgabeeinrichtung für allergene Partikel einer zweiten Art vorgesehen sind, wobei beide Abgabeeinrichtungen voneinander beabstandet sind. Die Abgabeeinrichtungen sind im Bereich einer Decke der Kammer angeordnet und so orientiert, dass die allergenen Partikel der ersten Art ausschließlich in einen ersten Korridor und die allergenen Partikel der zweiten Art ausschließlich in einen zweiten Korridor innerhalb des Raumes abgegeben werden, wobei die beiden Korridore zumindest über einen vorgegebenen Erstreckungsbereich nicht überlappen.

Auch hier kann es vorteilhaft sein, bei der Vorrichtung eine Luftzuführung im Bereich einer Decke des Raumes vorzusehen und eine Luftabsaugung im Bereich des Bodens des Raums, wobei Luftzuführung und Luftabsaugung derart ausgebildet und positioniert sind, dass zumindest im Bereich der Abgabeeinrichtungen eine Luftströmung mit einer Vorzugsrichtung erzeugt ist, die in Richtung der Schwerkraft weist.

In der nachfolgenden Beschreibung werden ausschließlich Ausführungsformen mit nicht mehr als zwei Abgabeeinrichtungen für unterschiedliche allergene Partikel beschrieben. Es versteht sich jedoch, dass erfindungsgemäß zwei oder mehr Abgabeeinrichtungen für allergene Partikel verschiedener Art vorgesehen sein können.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: einen Querschnitt durch eine Kammer; und
- Fig. 2: einen Längsschnitt durch die Kammer von Fig. 1.

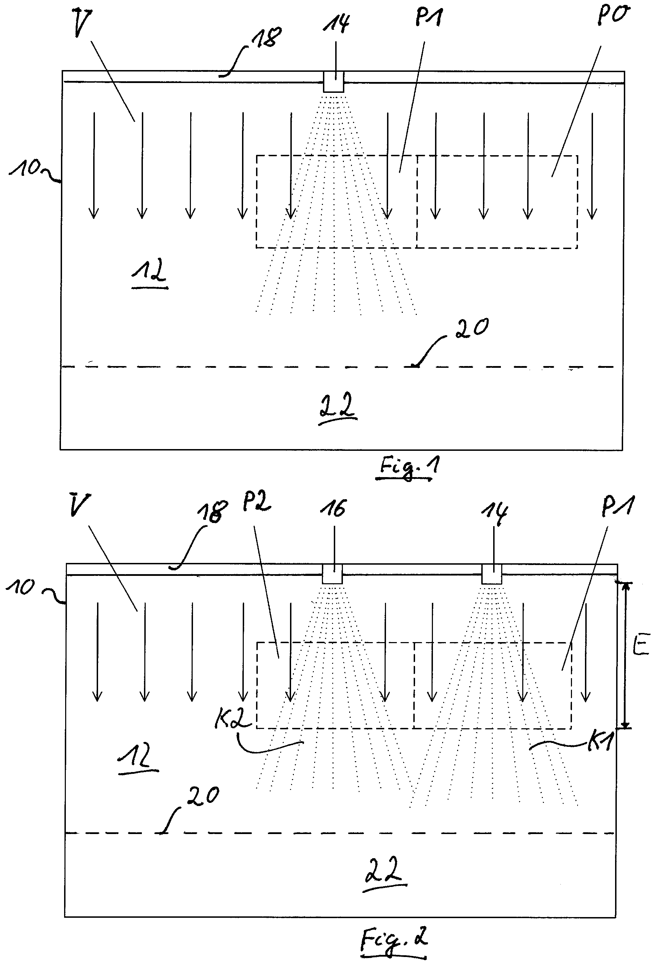

Die Fig. 1 und 2 zeigen in stark vereinfachter schematischer Darstellung einen Querschnitt und einen Längsschnitt durch eine Kammer 10, die insbesondere mobil ausgebildet ist, beispielsweise in Form eines Containers, und in deren Inneren ein Raum 12 vorgesehen ist. Der Raum 12 ist begehbar und weist im Bereich seiner Decke zumindest eine erste Abgabeeinrichtung 14 für allergene Partikel einer ersten Art und eine zweite Abgabeeinrichtung 16 für allergene Partikel einer zweiten Art auf, wobei die beiden Abgabeeinrichtungen 14 und 16 voneinander beabstandet sind. Bei den Abgabeeinrichtungen kann es sich um solche handeln, wie sie in der deutschen Patentanmeldung DE 10 2014 116 694.9 beschrieben sind, deren Inhalt durch Bezugnahme zum Inhalt der vorliegenden Anmeldung gemacht wird und deren Offenbarung in die hier vorliegende Anmeldung eingeschlossen ist.

Wie Fig. 2 verdeutlicht, sind die beiden Abgabeeinrichtungen 14 und 16 im Bereich der Decke des Raumes so angeordnet und orientiert, dass die allergenen Partikel der ersten Art ausschließlich in einen ersten Korridor K1 abgegeben werden und die allergenen Partikel einer zweiten Art durch die zweite Abgabeeinrichtung 16 ausschließlich in einen zweiten Korridor K2 innerhalb ein und desselben Raumes 12 abgegeben werden. Bei dem dargestellten Ausführungsbeispiel sind die Korridore ausgehend von den Abgabeeinrichtungen kegelförmig ausgebildet und die beiden kegelförmigen Korridore K1 und K2 überlappen über einen vorgegebenen Erstreckungsbereich E ausgehend von den Abgabeeinrichtungen 14 und 16 nicht. Auch sind zwischen den Korridoren K1 und K2 keine Trennwände oder dgl. vorgesehen. Hierbei ist der Erstreckungsbereich E ausgehend von den Abgabeeinrichtungen so gewählt, dass Patienten, die innerhalb des Raumes 12 auf vorgegebenen Sitzgelegenheiten sitzen, sich mit ihrem Oberkörper innerhalb des Erstreckungsbereiches E befinden. Auf diese Weise ist ausgeschlossen, dass Patienten gleichzeitig Partikel unterschiedlicher Art inhalieren. Ein Überlappen der Korridore K1 und K2 außerhalb des vorgegebenen Erstreckungsbereiches E ist insofern unschädlich, da außerhalb des Erstreckungsbereiches E keine Inhalation durch einen Patienten erfolgt.

Zur Unterstützung der Abgabe der allergenen Partikel ausschließlich in ihren zugeordneten Korridoren K1 und K2 ist im Bereich der Decke des Raumes 12 eine stark schematisch dargestellte Luftzuführung 18 vorgesehen, die zumindest im Bereich der Abgabeeinrichtungen 14 und 16 und bevorzugt im gesamten Raumbereich eine Luftströmung mit einer Vorzugsrichtung V erzeugt, die in Richtung der Schwerkraft weist. Hierbei kann die Luftzuführung 18 im Bereich der Deck grundsätzlich auf verschiedene Weise ausgebildet sein, beispielsweise durch Luftkanäle, die mit Luftauslassöffnungen versehen sind.

Zur Luftabsaugung der Raumluft ist in dem Raum 12 ein Gitterboden 20 vorgesehen, unter dem eine Luftabsaugung 22 angeordnet ist, mit der die Raumluft und auch die mit allergenen Partikeln versehene Luft abgesaugt wird.

Wie Fig. 2 verdeutlicht, sind die Korridore K1 und K2 in vertikaler Richtung nach unten ausgerichtet, wobei die Mittelachsen der Korridore K1 und K2 im Wesentlichen parallel zueinander ausgerichtet sind. Die Luftströmung mit Vorzugsrichtung vertikal nach unten, d.h. in Richtung der Schwerkraft, wird bevorzugt als im Wesentlichen laminare Luftströmung erzeugt, wobei die Luft insbesondere zumindest entlang zweier paralleler Wände des Raumes von der Decke eingebracht und vom Boden abgesaugt werden kann.

Erfindungsgemäß kann mit den beschriebenen Verfahren sowie mit der beschriebenen Vorrichtung innerhalb ein und desselben Raumes 12 ein definierter Partikelraum P1 geschaffen werden, in dem sich ausschließlich Partikel einer ersten Art befinden, die von der ersten Abgabeeinrichtung 14 in den Korridor K1 abgegeben worden sind. Neben diesem definierten Partikelraum P1 kann zu einer oder zu mehreren Seiten ein garantiert partikelfreier Raum P0 vorgesehen werden, so dass innerhalb ein und desselben Raumes 12 ein Patient im Partikelraum P1 exponiert werden kann, während ein anderer Patient im partikelfreien Raum P0 nicht exponiert wird.

Gleichzeitig ist es möglich, neben dem Partikelraum P1 einen definierten Partikelraum P2 vorzusehen, indem sich ausschließlich allergene Partikel einer zweiten Art befinden, die von der zweiten Abgabeeinrichtung 16 in den Korridor K2 abgegeben worden sind. Es liegen somit innerhalb ein und desselben Raumes 12 ohne Trennwände, Abtrennungen oder Barrieren ein oder mehrere spezifische Bereiche mit Partikeln vor, während es im gleichen Raum 12 spezifische Bereiche mit entweder keinen Partikeln oder aber anderen Partikeln gibt.

Zur Verwendung in den vorstehend beschriebenen Verfahren eignen sich grundsätzlich beliebige Partikel, insbesondere allergene Partikel, beispielsweise natürliche oder vorbehandelte Pollen.

## Patentansprüche

1. Verfahren zur kontrollierten Abgabe von allergenen Partikeln zumindest einer ersten und einer zweiten Art in einen Raum (12), wobei die allergenen Partikel der ersten Art an einer ersten Abgabestelle und die allergenen Partikel der zweiten Art an einer zweiten Abgabestelle in den Raum (12) abgegeben werden, die von der ersten Abgabestelle beabstandet ist, wobei die allergenen Partikel der ersten Art ausschließlich in einen ersten Korridor (K1) und die allergenen Partikel der zweiten Art ausschließlich in einen zweiten Korridor (K2) innerhalb des Raumes abgegeben werden, wobei die beiden Korridore zumindest über einen vorgegebenen Erstreckungsbereich (E) nicht überlappen.

2. Verfahren nach Anspruch 1,
**dadurch gekennzeichnet, dass**
die Korridore (K1, K2) in vertikaler Richtung nach unten ausgerichtet werden.

3. Verfahren nach Anspruch 1 oder 2,
**dadurch gekennzeichnet, dass**
Mittelachsen der Korridore (K1, K2) im Wesentlichen parallel zueinander ausgerichtet werden.

4. Verfahren nach zumindest einem der vorstehenden Ansprüche,
**dadurch gekennzeichnet, dass**
in dem Raum (12) zumindest im Bereich der Abgabestellen eine Luftströmung mit einer Vorzugsrichtung (V) erzeugt wird, die in Richtung der Schwerkraft weist.

5. Verfahren nach Anspruch 4,
**dadurch gekennzeichnet, dass**
die Luftströmung als im Wesentlichen laminare Luftströmung erzeugt wird.

6. Verfahren nach zumindest einem der vorstehenden Ansprüche,
**dadurch gekennzeichnet, dass**
Luft aus dem Raum (12) durch eine Bodenabsaugung (22) abgesaugt wird, die insbesondere zumindest entlang zweier paralleler Wände des Raums verläuft.

7. Verfahren nach zumindest einem der vorstehenden Ansprüche,
**dadurch gekennzeichnet, dass**
Luft in den Raum (12) durch eine Luftzuführung (18) eingebracht wird, die insbesondere entlang zweier paralleler Wände des Raums verläuft.

8. Verfahren nach zumindest einem der Ansprüche 4 - 7,
**dadurch gekennzeichnet, dass**
die Partikel in Richtung der Schwerkraft mit einer Geschwindigkeit in ihren Korridor (K1, K2) abgegeben werden, die größer ist als die Geschwindigkeit der Luftströmung.

9. Vorrichtung zur Durchführung des Verfahrens nach zumindest einem der vorstehenden Ansprüche, umfassend:
eine insbesondere mobile Kammer (10), in deren Inneren ein Raum (12) vorgesehen ist,
eine erste Abgabeeinrichtung (14) für allergene Partikel einer ersten Art und eine zweite Abgabeeinrichtung (16) für allergene Partikel einer zweiten Art, die von der ersten Abgabeeinrichtung (14) beabstandet ist,
wobei die Abgabeeinrichtungen (14, 16) im Bereich einer Decke des Raumes (12) angeordnet und so orientiert sind, dass die allergenen Partikel der ersten Art ausschließlich in einen ersten Korridor (K1) und die allergenen Partikel der zweiten Art ausschließlich in einen zweiten Korridor (K2) innerhalb des Raumes (12) abgegeben werden, wobei die beiden Korridore (K1, K2) zumindest über einen vorgegebenen Erstreckungsbereich (E) nicht überlappen.

10. Vorrichtung nach Anspruch 9,
**gekennzeichnet durch**
eine Luftzuführung (18), die im Bereich einer Decke des Raums (12) verläuft, und eine Luftabsaugung (22) im Bereich des Bodens (20) des Raums (12), wobei Luftzuführung und Luftabsaugung derart ausgebildet und positioniert sind, dass zumindest im Bereich der Abgabeeinrichtungen (14, 16) eine Luftströmung mit einer Vorzugsrichtung (V) erzeugt ist, die in Richtung der Schwerkraft weist.
